# EUROPEAN PATENT SPECIFICATION

(11) **EP 3 692 375 B1**
(45) Date of publication and mention of the grant of the patent: **02.12.2026**
(21) Application number: 18779400.3
(22) Date of filing: 05.10.2018
(51) Int. Cl.: G01N 33/92, A61M 16/04, G01N 21/35

(54) **FETAL LUNG MATURITY TEST**
REIFEBESTIMMUNG DER LUNGE VON FÖTEN
TEST DE MATURITÉ PULMONAIRE DU F TUS

(30) Priority: 06.10.2017 EP 17195178
(43) Date of publication of application: 12.08.2020
(73) Proprietor: Sime Diagnostics Ltd., London W1U 7LP (GB)
(72) Inventor: SCHOUSBOE, Peter, 5220 Odense (DK); VERDER, Henrik, 2880 Bagsværd (DK)
(74) Representative: Høiberg P/S
(86) International application number: PCT/EP2018/077090
(87) International publication number: WO 2019/068848

(56) References cited:
- WO-A1-2014/191406
- WO-A1-92/19232
- KAN-ZHI LIU ET AL: "Comparison of infrared spectroscopic and fluorescence depolarization assays for fetal lung maturity", AMERICAN JOURNAL OF OBSTETRICS AND GYNECOLOGY, vol. 183, no. 1, 1 July 2000 (2000-07-01), pages 181 - 187, XP055132528, ISSN: 0002-9378, DOI: 10.1067/mob.2000.105345
- PENEFF P ET AL: "Determination of lecithin and sphingomyelin (L/S ratio) in gastric and/or pharyngeal aspirates of the newborn", MINERVA PEDIAT, TORINO, IT, vol. 31, no. 11, 15 June 1979 (1979-06-15), pages 835 - 840, XP009179427, ISSN: 0026-4946
- LIU ET AL: "Rapid determination of fetal lung maturity from infrared spectra of amniotic fluid", AMERICAN JOURNAL OF OBSTETRICS & GYNECO, MOSBY, ST LOUIS, MO, US, vol. 178, no. 2, 1 February 1998 (1998-02-01), pages 234 - 241, XP005437844, ISSN: 0002-9378, DOI: 10.1016/S0002-9378(98)80006-9
- PETER A BARR, PATRICIA A JENKINS, J DAVID BAUM: "Lecithin/sphingomyelin ratio in hypopharyngeal aspirate of newborn infants", ARCHIVES OF DISEASE IN CHILDHOOD, vol. 50, 1 January 1975 (1975-01-01) - 1 January 1975 (1975-01-01), ENGLAND, pages 856 - 861, XP055433344, DOI: 10.1136/adc.50.11.856
- H VERDER ET AL: "Lamellar body counts on gastric aspirates for prediction of respiratory distress syndrome", ACTA PAEDIATRICA, vol. 100, no. 2, 4 October 2010 (2010-10-04), GB, pages 175 - 180, XP055132365, ISSN: 0803-5253, DOI: 10.1111/j.1651-2227.2010.02010.x
- ARRØE M ET AL: "Prediction of respiratory distress syndrome by the microbubble stability test on gastric aspirates in newborns of less than 32 weeks' gestation", ACTA PAEDIATRICA, vol. 92, no. 6, 1 January 2003 (2003-01-01), GB, pages 728 - 733, XP055433062, ISSN: 0803-5253, DOI: 10.1080/08035250310002597
- HENRIK VERDER ET AL: "Rapid test for lung maturity, based on spectroscopy of gastric aspirate, predicted respiratory distress syndrome with high sensitivity", ACTA PAEDIATRICA, vol. 106, no. 3, 20 December 2016 (2016-12-20), GB, pages 430 - 437, XP055433066, ISSN: 0803-5253, DOI: 10.1111/apa.13683
- VERDER HENRIK ET AL: "Early Surfactant Guided by Lamellar Body Counts on Gastric Aspirate in Very Preterm Infants", NEONATOLOGY, vol. 104, no. 2, 9 July 2013 (2013-07-09) - 9 July 2013 (2013-07-09), pages 116 - 122, XP009179440, ISSN: 1661-7800, [retrieved on 20130709], DOI: 10.1159/000351638

## Description

### Technical field

The present invention relates to methods for diagnosing Respiratory Distress Syndrome of newborns.

### Background

Surfactant deficiency at birth and development of Respiratory Distress Syndrome of newborn (RDS) is the most important cause of morbidity and mortality in preterm infants.

### Respiratory Distress Syndrome

Respiratory Distress Syndrome (RDS) also called idiopathic respiratory distress syndrome (IRDS) or neonatal respiratory distress syndrome [1], and hyaline membrane disease (HMD), is a syndrome in premature infants caused by developmental insufficiency of surfactant production and structural immaturity in the lungs. It can also be the result of genetic causes, challenging production of surfactant associated proteins. RDS affects about 1% of newborn infants and is the single leading cause of death and morbidity in preterm infants [2]. The incidence decreases with advancing gestational age, from about 50% in babies born at 26-28 weeks, to about 25% at 30-31 weeks. The syndrome is more frequent in infants of diabetic mothers, in the second born of premature twins, and in infants born under induced labours or caesarean sections.

The onset of RDS is shortly after birth, and is manifested by tachypnea, tachycardia, chest wall retractions (recession), expiratory grunting, nasal flaring and cyanosis during breathing efforts. As the disease progresses, the newborn may develop ventilatory failure (rising carbon dioxide concentrations in the blood), and prolonged cessations of breathing ("apnea"). Whether treated or not, the clinical course for the acute disease lasts for about 2 to 5 days. During the first days the condition of the patient often worsens and requires more support. Despite major advances in care, RDS remains the most common single cause of death in the first month of life. Complications include metabolic disorders (acidosis, low blood sugar), patent ductus arteriosus, low blood pressure, chronic lung changes, and intracranial haemorrhage. The disease is frequently complicated by prematurity and its accompanying defects in other organ functions.

The characteristic histopathology seen in babies who die from RDS was the source of the name "hyaline membrane disease". Waxy-appearing layers of hyaline membrane line the collapsed alveoli of the lung. In addition, the lungs show bleeding, over-distention of airways and damage to the lining cells.

Moderate to severe cases of RDS will progress if the condition is not treated. Early nasal continuous positive airway pressure (nCPAP) decreases or halts the progression so that mechanical ventilation (MV) can be avoided in many cases [3-5].

In addition, about half of infants with RDS treated with nCPAP need surfactant supplementation to stop the progression [6,7] as the lungs of infants with RDS are developmentally deficient of surfactant. Similarly, about 50% of infants treated with MV need surfactant [8]. These infants, in contrast to infants treated with nCPAP, often require more doses of surfactant for a sustained response [6].

### Surfactant

Surfactant is a surface-active lipoprotein complex produced by specialized lung cells called Type II cells or Type II pneumocytes. The proteins and lipids that comprise the surfactant have both a hydrophilic region and a hydrophobic region. By adsorbing to the air-water interface of alveoli with the hydrophilic head groups in the water and the hydrophobic tails facing towards the air, the main lipid component of surfactant, dipalmitoylphosphatidylcholine (DPPC), reduces surface tension. In addition to DPPC which constitutes about 40%, the surfactant complex comprises about 40% other phospholipids, about 5% surfactant-associated proteins (SP-A, B, C and D) and additionally cholesterol and trace amounts of other substances.

The function of the surfactant complex is to increase pulmonary compliance, to prevent atelectasis (collapse of the lung) at the end of expiration and to facilitate recruitment of collapsed airways.

Surfactant helps prevent collapse of the terminal air-spaces throughout the normal cycle of inhalation and exhalation. The surfactant is packaged by the cell in structures called lamellar bodies, and extruded into the air-spaces. The lamellar bodies subsequently unfold into a complex lining of the air-space. This layer reduces the surface tension of the fluid that lines the air-space. Surface tension is responsible for approximately 2/3 of the elastic recoil forces. In the same way that a bubble will contract to give the smallest surface area for a given volume, at the air/water interface the liquid surface will tend towards being as small as possible, thereby causing the air-space to contract. By reducing surface tension, surfactant prevents the air-spaces from completely collapsing on exhalation. In addition, the decreased surface tension allows re-opening of the air-space with a lower amount of force. Therefore, without adequate amounts of surfactant, the air-spaces collapse and are very difficult to expand. Microscopically, a surfactant deficient lung is characterized by collapsed air-spaces alternating with hyper-expanded areas, vascular congestion and, in time, hyaline membranes. Hyaline membranes are composed of fibrin, cellular debris, red blood cells, rare neutrophils and macrophages. They appear as an eosinophilic, amorphous material, lining or filling the air spaces and blocking gas exchange. As a result, blood passing through the lungs is unable to pick up oxygen and unload carbon dioxide. Blood oxygen levels fall and carbon dioxide rises, resulting in rising blood acid levels and hypoxia. Structural immaturity, as manifest by decreased number of gas-exchange units and thicker walls, also contributes to the disease process. Therapeutic oxygen and positive-pressure ventilation, while potentially life-saving, can also damage the lung. The current diagnosis is based on the clinical condition supplemented by chest x-ray, which demonstrates decreased lung volumes (bell-shaped chest), a small (0.5-1 mm), discrete, uniform infiltrate (sometimes described as a "ground glass" appearance) that involves all lobes of the lung, and air-bronchograms (i.e. the infiltrate will outline the larger airways passages which remain air-filled). In severe cases, this becomes exaggerated until the cardiac borders become unapparent (a 'white-out' appearance).

In pregnancies of greater than 30 weeks, the fetal lung maturity may be tested by sampling the amount of surfactant in the amniotic fluid by amniocentesis, wherein a sampling syringe needle is inserted through the mother's abdomen and uterus. Several tests are currently available that correlate the production of surfactant. One of the most important tests involves measurement of the concentration ratio between the phospholipids lecithin and sphingomyelin, the so called "L/S ratio". An L/S ratio of less than about 2.0 was previously found to be an indication that the fetal lungs are deficient [9].

A therapeutic standard procedure in very preterm infants has been to start with nCPAP, and, as surfactant is better administered early than late [6,10,11], to give surfactant during a short intubation as soon as clinical symptoms and an increasing oxygen requirement indicate moderate to severe RDS [6,12]. This so-called INSURE (intubation surfactant extubation) procedure is now widely used and has resulted in diminished use of MV [10] and a decreased incidence of bronchopulmonary dysplasia (BPD) [11,13]. Many infants are still given surfactant relatively late - typically the median age at treatment is 5 h [4]. Additionally, common clinical criteria for identification of infants with RDS in need of surfactant and timing of this treatment are missing [14]. Consequently, there is a need for a rapid quantitative method to identify which of the very preterm infants who are at risk of failing nCPAP and who therefore should receive surfactant at an early stage. Prophylactic surfactant treatment as an alternative has proven suboptimal [13]. Preferably the diagnosis and treatment should start immediately after birth. The present state of the art methods requires time-consuming laboratory tests to be performed thus delaying diagnosis and onset of medication for those in need thereof [15].

### Lamellar bodies

Lamellar bodies are also termed lamellar granules, membrane-coating granules (MCGs), keratinosomes or Odland bodies. They are lipid storage and secretory organelles found in type II alveolar cells in the lungs, and in keratinocytes in the skin. They are oblong structures, appearing about 300-400 nm in length and 100-150 nm in width in transmission electron microscopy images. Lamellar bodies fuse with the cell membrane and release pulmonary surfactant into the extracellular space. They are surrounded by a membrane and contain multilamellar lipid membranes. They may also contain apolipoproteins and lytic enzymes and have an acidic pH. Under normal physiological conditions, their main function is the supply of extracellular domains with specialised lipid components related to a specialised function. The lamellar bodies of the lung epithelium are the storage form of lung surfactant. They provide a monomolecular lipid film of dipalmitoyl phosphatidylcholine on the surface of alveoli to lower surface tension necessary for optimal gas exchange. They also provide a hydrophobic protective lining against environmental influences. Lamellar bodies are also found in other cell types of the respiratory system, for example the mucosa of the nose and the bronchia.

The gastrointestinal tract, the tongue papillae, the oral epithelium and mucosal cells of the stomach also contain lamellar bodies. Phosphatidylcholine is the major phospholipid of lamellar bodies in mucosa cells of the stomach, providing a hydrophobic protective lipid film against the tissue-damaging activities of gastric juice.

The hydrophobic water-protective barrier of the skin also originates from lamellar bodies secreted by epithelial cells, and consists mainly of neutral lipids. Lamellar bodies also occur in mesodermal cell layers of sliding surfaces to provide joint lubrication, as well as in the peritoneum, the pericardium and the pleural mesothelium.

Lamellar bodies have also been found to accumulate in several pathological conditions, such as atherosclerosis, Niemann-Pick disease. The fact that lysosomal lamellar bodies are absent in the normal intima of the aortic wall, but appear in cells in fatty streaks might indicate that their formation is relevant to the pathogenetic mechanisms which become involved in the development of atherosclerosis.

WO 2014/191406 relates to diagnosis of respiratory distress syndrome (RDS) and discloses determining the amount of sphingomyelin and lecithin in body fluid sample using an analysis unit, obtaining a ratio of these compounds and correlating the ratio with a control ratio.

Kan-Zhi Liu et al.: "American Journal of Obstetrics And Gynecology, vol. 183, no. 1, 1 July 2000 (2000-07-01), Pages 181-187, relates to prediction of fetal lung maturity and discloses measurement of phospholipids using the lecithin/sphingomyelin ratio by means of thin-layer chromatography.

Peneff P et al.:, Minerva Pediat, Torino, IT, vol. 31, no. 11, 15 June 1979 (1979-06-15), pages 835-840, relates to the prognosis of fetal pulmonary maturity and discloses determination of lecithin and sphingomyelin in the gastric and/or pharyngeal aspirate of the newborn.

Liu et al.: American Journal of Obstetrics & Gyneco, Mosby, St Louis, MO, US, vol. 178, no. 2, 1 February 1998, (1998-02-01), pages 234-241, relates to determination of fetal lung maturity and discloses measurement of this by infrared spectra of amniotic fluid.

Peter A Barr, Patricia A Jenkins, J David Baum: Archives of Disease in Childhood, vol. 50 , 1 January 1975 (1975-01-01), pages 856-861, relates to samples flushed from cathethers or dissolved in saline, chloroform extraction and analysis by thin layer chromatography.

Henrik Verder et al.: Acta Paediatrica, vol. 100, no. 2, 4 October 2010 (2010-10-04), pages 175-180, discloses lamellar body counts on gastric aspirate for prediction of development of RDS in infants.

Arrøe M et al.: Acta Paediatrica, vol. 92, no. 6, 1 January 2003 (2003-01-01), pages 728-733, discloses that the determination of the lecithin/sphingomyelin ratio (L/S ratio) is independent of volume fluctuations.

Henrik Verder et al.:, Acta Paediatrica, vol. 106, no. 3, 20 December 2016 (2016-12-20), pages 430-437, discloses measurement by mass spectrometry and infrared spectroscopy of the concentrations of di-palmitoyl-phosphatidyl-choline and sphingomyelin in gastric aspirates from premature infants.

WO 92/19232 relates to assessment of fetal lung maturity and discloses measurement of the lecithin/sphingomyelin ratio in amniotic fluid.

Verder Henrik et al.: Neonatology, vol. 104, no. 2, pages 116-122, relates to the prediction of respiratory distress syndrome in newborns and discloses measuring lamellar body counts (LBC) in gastric aspirate.

### Description of drawings

Fig. 1 Left panel: frozen and thawed gastric aspirate samples show mucus-like, flocculent material, mainly composed of phospholipids and proteins. Right panel: fresh gastric aspirate samples do not show flocculent material.
Fig. 2 Analysis of crude GAS and purified LB fractions analysed by MS show high correlations between L/S ratio and RDS. The horizontal line shows an appropriate cut-off value (3.0) for determining whether a newborn suffers from RDS. Sensitivity of the method is 91%, and specificity is 81%.
Fig. 3 Upper panel: electron microscopy of precipitated lamellar bodies from GAS at birth. Lower panel: lamellar bodies from GAS in large electron microscopy magnification.

### Summary

The invention is as defined in the claims. Any subject matter falling outside the scope of the claims is provided for information purposes only.

Herein is provided a method for diagnosing Respiratory Distress Syndrome (RDS) in a subject, the method comprising the steps of:
i) providing a sample from said subject, wherein the sample is an amniotic fluid sample, a blood sample, an oropharyngeal secretion sample or a gastric aspirate sample, preferably the sample is a gastric aspirate sample or an oropharyngeal secretion;
ii) homogenising and diluting said sample in a first volume of a first solution, thereby obtaining a homogenous sample;
iii) centrifuging the sample of step ii) to obtain a pellet comprising lamellar bodies, and a supernatant;
iv) discarding the supernatant and resuspending the pellet in a second volume of a second solution, thereby obtaining a sample for analysis;
v) determining the amount of a first compound selected from lecithin and saturated lecithin and optionally the amount of sphingomyelin in the sample for analysis, using analysis means;
vi) obtaining a concentration of the first compound and/or a ratio between the first compound and sphingomyelin;
vii) correlating the concentration of vi) with a control value, wherein a concentration equal to or lower than the control value is indicative of the subject having Respiratory Distress Syndrome (RDS); and/or correlating the ratio of vi) with a control ratio, wherein a ratio equal to or lower than the control ratio is indicative of the subject having Respiratory Distress Syndrome (RDS).

Also provided is surfactant for use in the treatment of Respiratory Distress Syndrome (RDS) in a newborn individual, wherein the surfactant is administered to a subject having an increased likelihood of RDS as determined by the method comprising the steps of:
i) providing a sample from said newborn individual, wherein the sample is an amniotic fluid sample, a blood sample, an oropharyngeal secretion sample or a gastric aspirate sample;
ii) diluting and homogenising said sample in a first volume of a first solution, thereby obtaining a homogenous sample;
iii) centrifuging the homogenous sample to obtain a pellet comprising lamellar bodies and a supernatant;
iv) discarding the supernatant and resuspending the pellet in a second volume of a second solution, thereby obtaining a sample for analysis;
v) determining the amount of a first compound selected from lecithin and saturated lecithin and optionally the amount of sphingomyelin in the sample for analysis, using analysis means;
vi) obtaining a concentration of the first compound and optionally a ratio between the first compound and sphingomyelin; and
vii) correlating the concentration of vi) with a control value, wherein a concentration equal to or lower than the control value is indicative of the subject having Respiratory Distress Syndrome (RDS); and/or correlating the ratio of vi) with a control ratio, wherein a ratio equal to or lower than the control ratio is indicative of the subject having Respiratory Distress Syndrome (RDS),
optionally wherein steps i) to vii) are performed as described elsewhere herein.

Herein is also provided a computer implemented method for diagnosing RDS based on spectral data acquired from a sample obtained from a subject, wherein the sample is an amniotic fluid sample, a blood sample, an oropharyngeal secretion sample or a gastric aspirate sample, the method comprising the steps of:
i) determining the activity and/or concentration of a first compound selected from lecithin and saturated lecithin and optionally of sphingomyelin in said sample by acquiring spectral data for the sample,
ii) optionally calculating a ratio between the activities and/or concentrations of sphingomyelin and of the first compound,
iii) correlating said concentration with a control value and optionally correlating said ratio with a control ratio, wherein a concentration equal to or lower than the control value and/or a ratio equal to or lower than the control ratio is indicative of RDS of the subject,
wherein the method further comprises the features as disclosed elsewhere herein.

Also provided is a computer program product having a computer readable medium, said computer program product suitable for diagnosing RDS of a subject based on spectral data acquired from a sample obtained from said subject, wherein the sample is an amniotic fluid sample, a blood sample, an oropharyngeal secretion sample or a gastric aspirate sample, said computer program product comprising means for carrying out all the steps of the method as defined elsewhere herein.

### Detailed description

### Definitions

The references to methods of treatment in this description are to be interpreted as references to the compounds, pharmaceutical compositions and medicaments of the present invention for use in a method for the treatment of the human or animal body by therapy.

### Analysis means

The term 'analysis means' as used herein refers to an instrument capable of detecting the physical property of a molecule or group of molecules. In one embodiment the analysis means is a FTIR spectrometer. Preferably the analysis means is an FTIR spectrometer capable of performing measurements in very small sample volumes such as down to 1 µL.

### Mid-IR

The term Mid-IR or Mid wavelength infrared, also called intermediate infrared (IIR) and mid-red FTIR spectroscopy as used herein refers to light having a wavelength of between about 3 to about 50µm.

### Premature infant

The term premature infant as used herein refers to an infant born before or up to 37 weeks into the pregnancy.

### Respiratory distress syndrome of newborn

The term "Respiratory Distress Syndrome" (RDS) as used herein refers to the term as understood by those of skill in the art. RDS may also be defined as P22.0 of WHO's ICD-10 disease classification. The abbreviation RDS stands for Respiratory Distress Syndrome.

### Method of diagnosing RDS

It is essential for the success of treatment of respiratory distress syndrome (RDS) in newborn premature infants, to rapidly assess the status of the development of the lungs of the infant. This can be performed by measuring the amount of lecithin and sphingomyelin. However, the methods known to date require sample preparation which is time consuming and thus delays commencement of medication by surfactant. The methods of the current state of the art furthermore typically rely on samples which can be heterogeneous, thus affecting the reproducibility - and consequently the reliability of the diagnosis. Moreover, since the samples are derived from newborns, it is important that the volume required for analysis is as small as possible, which is a challenge for conventional methods.

The present inventors have found that measuring the amount of lecithin and optionally of sphingomyelin in the lamellar bodies contained in a sample obtained from the newborn can be used to reliably predict RDS. The methods are fast and require but minute sample volumes and provide reliable and reproducible results.

The present inventors have thus addressed the above problems and found that it is possible to analyse very low volume samples from e.g. gastric aspirate samples obtained from the newborn individual and to determine the concentration of lecithin or saturated lecithin and optionally the ratio between lecithin and sphingomyelin in the lamellar bodies contained in the samples, without time-consuming laboratory sample preparations.

The present methods are thus based on precipitating said lamellar bodies, removing at least part of the cellular debris from the samples, and determining the concentration of lecithin or saturated lecithin and or the L/S ratio with analysis means. The inventors have surprisingly found that the L/S ratio as determined by the present methods can reproducibly and reliably be used to diagnose RDS in such a short time frame that the methods are well suited for point of care units.

Thus in a main aspect the present invention concerns a method for diagnosing Respiratory Distress Syndrome (RDS), the method comprising the steps of:
i) providing a sample from said subject, wherein the sample is an amniotic fluid sample, a blood sample, an oropharyngeal secretion or a gastric aspirate sample;
ii) diluting and homogenising said sample in a first volume of a first solution, thereby obtaining a homogenous sample;
iii) centrifuging the sample of step ii) to obtain a pellet comprising lamellar bodies, and a supernatant;
iv) discarding the supernatant and resuspending the pellet in a second volume of a second solution, thereby obtaining a sample for analysis;
v) determining the amount of a first compound selected from lecithin and saturated lecithin and optionally the amount of sphingomyelin in the sample for analysis, using analysis means;
vi) obtaining a concentration of the first compound and/or a ratio between the first compound and sphingomyelin;
vii) correlating the concentration of vi) with a control value, wherein a concentration equal to or lower than the control value is indicative of the subject having Respiratory Distress Syndrome (RDS); and/or correlating the ratio of vi) with a control ratio, wherein a ratio equal to or lower than the control ratio is indicative of the subject having Respiratory Distress Syndrome (RDS).

In a further aspect, a method of treatment of Respiratory Distress Syndrome (RDS) in a newborn individual is provided, comprising the steps of:
i) providing a sample such as an amniotic fluid sample, a blood sample, an oropharyngeal secretion or a gastric aspirate sample from said newborn individual;
ii) diluting and homogenising said sample in a first volume of a first solution, thereby obtaining a homogenous sample;
iii) centrifuging the homogenous sample to obtain a pellet comprising lamellar bodies and a supernatant;
iv) discarding the supernatant and resuspending the pellet in a second volume of a second solution, thereby obtaining a sample for analysis;
v) determining the amount of a first compound selected from lecithin and saturated lecithin and optionally the amount of sphingomyelin in the sample for analysis, using analysis means;
vi) obtaining a concentration of the first compound and optionally a ratio between the first compound and sphingomyelin;
vii) correlating the concentration of vi) with a control value and optionally the ratio of vi) with a control ratio, wherein a concentration equal to or less than the control value and/or a ratio equal to or less than the control ratio is indicative of Respiratory Distress Syndrome (RDS) of the subject;
viii) if the concentration of vi) is equal to or less than the control value and/or if the ratio of vi) is equal to or less than the control ratio, administering a therapeutically effective amount of surfactant to the newborn individual.

In a further aspect, a method of treatment of Respiratory Distress Syndrome (RDS) in a newborn individual is provided, comprising the steps of:
i) providing a gastric aspirate sample from said newborn individual;
ii) diluting and homogenising said sample in a first volume of a first solution, thereby obtaining a homogenous sample;
iii) centrifuging the homogenous sample to obtain a pellet comprising lamellar bodies and a supernatant;
iv) discarding the supernatant and resuspending the pellet in a second volume of a second solution, thereby obtaining a sample for analysis;
v) determining the amount of a first compound selected from lecithin and saturated lecithin and the amount of sphingomyelin in the sample for analysis, using analysis means;
vi) obtaining a ratio between the first compound and sphingomyelin;
vii) correlating the ratio of vi) with a control ratio, wherein a ratio differing from the control ratio is indicative of Respiratory Distress Syndrome (RDS) of the subject;
viii) if the ratio of vi) is less than the control ratio, administering a therapeutically effective amount of surfactant to the newborn individual.

In a clinical setting, the physician utilising the present methods may, based on the result of the diagnostic method, apply the method of exclusion to determine if the subject wherefrom the sample has been obtained, is suffering from RDS. If the result of the method indicates a concentration of lecithin or saturated lecithin equal to or less than 49.0 µmol/L, or if the result of the method indicates an L/S ratio significantly above a control ratio, the subject does not suffer from RDS. If the clinician determines that the condition of the subject is severe, but that the concentration of lecithin or the L/S ratio is significantly above a control ratio, the clinician can thus conclude that the subject is suffering from a critical condition other than RDS, and continue analysis and apply the appropriate treatment.

### Samples

The present methods may be performed on a sample selected from an amniotic fluid sample, a blood sample, an oropharyngeal secretion sample or a gastric aspirate sample. In one embodiment, the sample is an amniotic fluid sample. In another embodiment, the sample is an oropharyngeal secretion sample. In another embodiment, the sample is a gastric aspirate sample. In another embodiment, the sample is a blood sample.

If the sample is obtained from amniotic fluid, care should be taken to prevent contamination of the amniotic fluid. In one embodiment, the sample is obtained from a subject, such as a human being e.g. a female, such as a pregnant female.

The chances of collecting non-contaminated or essentially non-contaminated amniotic fluid are good in connection with caesarean sectioning. Thus in one embodiment the subject is a female human being, undergoing, or immediately about to undergo, caesarean sectioning. In a further embodiment the body fluid sample is amniotic fluid collected from the female human being, during or immediately subsequent to the caesarean sectioning.

As mentioned above, the present method allows for very small sample volumes. In one embodiment, the sample has a volume between 10 and 1000 µL, such as between 10 and 750 µL, such as between 20 and 500 µL, such as between 30 and 250 µL, such as between 40 and 125 µL, such as between 50 and 100 µL, such as between 60 and 90 µL, such as between 70 and 80 µL, such as 50 µL, 75 µL or 100 µL. In some embodiments, the sample has a volume less than 1000 µL, such as less than 900 µL, such as less than 800 µL, such as less than 700 µL, such as less than 600 µL, such as less than 500 µL, such as less than 400 µL, such as less than 300 µL, such as less than 200 µL, such as less than 100 µL, such as less than 90 µL, such as less than 80 µL, such as less than 70 µL, such as less than 60 µL, such as less than 50 µL. In specific embodiments, the volume of the sample is 50 µL, 75 µL or 100 µL.

Preferably, the sample is untreated prior to performing the present methods. Care should be taken however to try and obtain a sample which is as homogeneous as possible. Step ii) of the methods preferably comprises a step of homogenising the sample, as described below.

In particular, the sample is preferably not frozen prior to performing the present methods. The samples may if needed be stored at low temperatures for up to several weeks prior to analysis by the present methods, as storage has been found not to affect the phospholipid content as shown in the examples. For example, storage may be for 1 hour or more, such as 2 hours or more, such as 3 hours or more, such as 6 hours or more, such as 12 hours or more, such as 24 hours or more, such as 2 days or more, such as 3 days or more, such as 1 week or more, such as 2 weeks or more, such as 1 month. Storage is preferably at a temperature between 1 and 10°C, such as 2°C, 3°C, 4°C, 5°C, 6°C, 7°C, 8°C, 9°C or 10 °C, preferably at 4°C or 5°C.

### Homogenisation of the sample

In step ii), the sample is diluted and homogenised in a first solution. Homogenisation can be achieved as is known to the skilled person. For example, the sample may be placed on a vortex, thereby strongly stirring the sample. In some embodiments, the sample is diluted prior to and/or after homogenisation.

The sample is diluted in a first volume of a first solution, preferably a hypotonic solution. The first solution may be water, such as deionized water, or tap water. In one embodiment of the method, the first solution is deionized water. In another embodiment, the first solution is plain water, such as tap water. Without being bound by theory, it is hypothesized that the first solution lyses the cells, thereby facilitating precipitation of lamellar bodies in the next steps of the method.

The volume of the first solution can vary, since the sample is precipitated in later steps of the method. The volume of the first solution should preferably be at least equal to half the volume of the sample, such as at least equal to the volume of the sample, such as at least equal to twice the volume of the sample, such as at least equal to three times the volume of the sample, such as at least equal to four times the volume of the sample, such as at least equal to five times the volume of the sample, such as at least equal to 6 times the volume of the sample, such as at least equal to 7 times the volume of the sample, such as at least equal to 8 times the volume of the sample, such as at least equal to 9 times the volume of the sample, such as at least equal to 10 times the volume of the sample, or more. Thus in one embodiment, the ratio of the volume of the sample of step i) to the volume of the first solution used in step 2 is 1:0,5. In another embodiment the ratio of the sample of step i) to the volume of the first solution used in step ii) is 1:1. In another embodiment the ratio of the sample of step i) to the volume of the first solution used in step ii) is 1:2. In another embodiment the ratio of the sample of step i) to the volume of the first solution used in step ii) is 1:3. In another embodiment the ratio of the sample of step i) to the volume of the first solution used in step ii) is 1:4. In another embodiment the ratio of the sample of step i) to the volume of the first solution used in step ii) is 1:5. In another embodiment the ratio of the sample of step i) to the volume of the first solution used in step ii) is 1:6. In another embodiment the ratio of the sample of step i) to the volume of the first solution used in step ii) is 1:7. In another embodiment the ratio of the sample of step i) to the volume of the first solution used in step ii) is 1:8. In another embodiment the ratio of the sample of step i) to the volume of the first solution used in step ii) is 1:9. In another embodiment the ratio of the sample of step i) to the volume of the first solution used in step ii) is 1:10.

After adding the volume of the first solution to the sample of step i), the sample and the first solution are mixed and homogenized, e.g. by vortexing or pipetting as is known to the skilled person, until the mixture appears homogenous to the naked eye. A homogenous sample is thereby obtained.

### Precipitation of the lamellar bodies

In step iii) of the method, the homogenous sample obtained in step ii) is transferred to a centrifuge, and centrifugation is performed in order to obtain a pellet comprising lamellar bodies and a supernatant. In step iv), the supernatant is discarded.

Centrifugation is performed as is known in the art, at a force and for a duration sufficient to allow the lamellar bodies to be precipitated from the sample, so that a pellet is obtained comprising lamellar bodies. Without being bound by theory, centrifugation is thought to allow removal of substantially all or almost all the cellular debris.

Centrifugation may be performed at a force between 500 and 10000 g, such as between 1000 and 9000 g, such as between 2000 and 800 g, such as between 3000 and 7000 g, such as between 3500 and 6000 g, such as between 3750 and 5000 g, such as between 3750 and 4500 g, such as at about 4000 g. Centrifugation may be performed for a duration of 1 min to 10 min, such as 2 min to 9 min, such as 3 min to 8 min, such as 4 min to 7 min, such as 5 min to 6 min, such as 4 min, 5 min or 6 min.

For example, a centrifugation step of 4 minutes at 4000 g is suitable for performing the methods of the invention. Alternatively, the centrifugation may be for 2 minutes at 5000 g or more, for example 6000 g or more, for example 7000 g or more, for example 8000 g or more, for example 9000 g or more, for example 10000 g or more. The centrifugation may be for 10 minutes at 500 g or more, for example 1000 g or more, such as 2000 g or more, for example 3000 g or more, such as 4000 g or more, for example 5000 g or more, such as 6000 g or more, for example 7000 g or more, such as 8000 g or more, for example 9000 g or more, for example 10000 g.

Following centrifugation, the sample should now present two phases: a solid phase, or pellet, located at the bottom of the tube, and which may be invisible to the naked eye; and a liquid phase, or supernatant. The pellet comprises lamellar bodies from the sample in a concentrated form. The supernatant may also comprise a portion of lamellar bodies; however, the majority of lamellar bodies is preferably present in the pellet.

The supernatant is discarded as is known in the art. This may be done by pipetting the supernatant away, while being careful not to disturb the pellet, or it may be done by simply gently pouring the supernatant away, and optionally pipetting the remaining volume. The container in which the sample is comprised may be tipped gently and tapped gently on a piece of e.g. absorbing paper, in order to remove the small volumes of liquid which may remain on the walls of the container by gravity.

In some embodiments, the method is temperature-independent at least when performed in a temperature range between 20°C and 40°C.

### Analysis of the sample

Once the pellet is essentially free of supernatant, it is resuspended in a volume of a second solution, so that a sample for analysis is obtained. The second solution may be a hypotonic solution or a saline solution. The second solution may be water, such as deionized water, or tap water. In one embodiment of the method, the second solution is saline solution. In another embodiment, the second solution is deionized water. In a third embodiment, the second solution is plain water, such as tap water.

The volume of second solution to be added to the pellet depends on the analysis means used in step v), and will be described in more detail below.

The sample for analysis is used to determine the amount of a first compound selected from lecithin and saturated lecithin, and optionally the amount of sphingomyelin. In one embodiment, the sample for analysis is used to determine the amount of a first compound selected from lecithin and saturated lecithin, and the amount of sphingomyelin. Without being bound by theory, it is thought that the amounts determined in this step are representative of the amounts present in the original sample, i.e. the sample provided in step i), or are at least proportional to the amounts present in the original sample. Accordingly, the concentration of the first compound in the sample for analysis is preferably equal to or substantially equal to the concentration of the same in the sample provided in step i), i.e. the sample obtained from a subject. The ratio of the first compound to sphingomyelin in the sample for analysis is preferably equal to or substantially equal to the ratio of the same in the sample provided in step i), i.e. the sample obtained from a subject. Said ratio will herein be termed L/S ratio.

In some embodiments, it may be desirable to remove at least part or all of the second solution after resuspension or prior to analysis. This can be done for example by evaporation of at least part or all of the second solution. In some embodiments, the method thus comprises a step of drying the sample after resuspension and/or prior to determining the amount of the first compound and/or of sphingomyelin.

The volume of sample for analysis suitable for determining the amounts of the first compound and optionally of sphingomyelin may vary. In one embodiment, the volume of sample for analysis suitable for determining the amounts of the first compound and of sphingomyelin may vary.

In some embodiments, the sample for analysis may be transferred to a CaF₂ window. Removal of at least part of the second solution may thus aptly be performed at the time of transfer, for example if the CaF₂ window is at a high temperature allowing for evaporation, such as 80°C or more, such as 85°C, 86°C, 87°C, 88°C, 89°C, 90°C, 91°C, 92°C, 93°C, 94°C, 95°C or more. Part of the second solution may also have been evaporated prior to this step, and in some embodiments the method thus comprises two steps of removing at least part of the second solution prior to determining the amounts of the first compound and/or sphingomyelin.

In some embodiments, the sample for analysis is analysed using an infrared spectrometer. In a particular embodiment, the analysis means is a Fourier transformed infrared spectrometer (FTIR). Preferably, the amounts of sphingomyelin and/or of the first compound are determined in the mid-wavelength infrared range. Preferably, the amounts of sphingomyelin and of the first compound are determined in the mid-wavelength infrared range.

Suitable sample volumes for spectrometer analysis, in particular FTIR analysis, may be between 10 and 200 µL, such as between 25 and 175 µL, such as between 50 and 150 µL, such as between 75 and 125 µL, such as 100 µL, 75 µL, 50 µL, or 25 µL.

The present methods allow determination of the concentration of lecithin or saturated lecithin and optionally of the L/S ratio by measuring the amount of lecithin and saturated lecithin and optionally of sphingomyelin and lecithin or saturated lecithin, respectively. In some embodiments, the present methods allow determination of the L/S ratio by measuring the amount of sphingomyelin and lecithin, or by measuring the amount of sphingomyelin and saturated lecithin. The methods may also further include determining the amount of a third compound. In some embodiments, the third compound is phosphatidylglycerol.

The amount of a compound can be measured as a concentration or as an activity, as the person of skill is well aware of. In some embodiments of the disclosure, the amount of a compound is thus its concentration and/or activity.

Once the amounts of lecithin or saturated lecithin and/or sphingomyelin have been determined, the concentration of lecithin or saturated lecithin is calculated and compared with a control value, and/or the L/S ratio is calculated and compared with a control ratio, as described below. In some embodiments, once the amounts of sphingomyelin and/or lecithin or saturated lecithin have been determined, the L/S ratio is calculated and compared with a control ratio, as described below.

### Diagnostic

The concentration of lecithin or saturated lecithin in the lamellar bodies is compared in some embodiments to a control value. The control value is a threshold value, which corresponds substantially to the concentration of lecithin or saturated lecithin measured in subjects which do not suffer from RDS.

In some embodiments, the control value is 49.0 µmol/L ± 0.5 µmol/L, such as 49.0 µmol/L. A concentration of lecithin or saturated lecithin equal to or less than 49.0 µmol/L ± 0.5 µmol/L is indicative of the subject suffering from RDS. In this case, the subject may be treated as is known in the art, for example as described herein.

In some embodiments, the control value is between 45.0 µmol/L ± 0.5 µmol/L and 53 µmol/L ± 0.5 µmol/L, such as between 46.0 µmol/L ± 0.5 µmol/L and 52 µmol/L ± 0.5 µmol/L, such as between 47 µmol/L ± 0.5 µmol/L and 51 µmol/L ± 0.5 µmol/L, such as between 48 µmol/L ± 0.5 µmol/L and 50 µmol/L ± 0.5 µmol/L, such as 49.0 µmol/L ± 0.5 µmol/L.

Accordingly, if the concentration of lecithin or saturated lecithin in the lamellar bodies as measured by the methods disclosed herein is equal to or less than 55.0 µmol/L ± 0.5 µmol/L, such as less than 54.0 µmol/L ± 0.5 µmol/L, such as equal to or less than 53.0 µmol/L ± 0.5 µmol/L, such as equal to or less than 52.0 µmol/L ± 0.5 µmol/L, such as equal to or less than 51 µmol/L ± 0.5 µmol/L, such as equal to or less than 50.0 µmol/L ± 0.5 µmol/L, such as equal to or less than 49.0 µmol/L ± 0.5 µmol/L, the subject is classified as having or likely to have RDS. Preferably, the control value is 49.0 µmol/L ± 0.5 µmol/L, 50.0 µmol/L ± 0.5 µmol/L or 48.0 µmol/L ± 0.5 µmol/L.

In some embodiments, the L/S ratio in the sample is determined and compared to a control ratio. The control ratio is a threshold value, which corresponds substantially to the L/S ratio measured in subjects which do not suffer from RDS.

In some embodiments, the control ratio is between 1.0 and 2.5 ± 0.5. An L/S ratio smaller than the control ratio is indicative of the subject suffering from RDS. In this case, the subject may be treated as is known in the art, for example as described herein.

The control ratio in some embodiments is between 2.0 and 4.0 ± 0.5.

In some embodiments, the control ratio is between 1.0 and 3.0 ± 0.5, such as between 1.5 and 2.9 ± 0.5, such as between 2.0 and 2.8 ± 0.5, such as between 2.2 and 2.7 ± 0.5, such as between 2.4 and 2.6 ± 0.5, such as 2.5 ± 0.5 or 2.0 ± 0.5. In some embodiments, the control ratio is between 2.0 and 5.0 ± 0.5, such as between 2.2 and 4.5 ± 0.5, such as between 2.4 and 4.0 ± 0.5, such as between 2.6 and 3.5 ± 0.5, such as between 2.8 and 3.2 ± 0.5, such as 3 ± 0.5.

Accordingly, if the L/S ratio in the lamellar bodies as measured by the methods disclosed herein is equal to or less than 1.0 ± 0.5, such as equal to or less than 1.2 ± 0.5, such as equal to or less than 1.5 ± 0.5, such as equal to or less than 1.7 ± 0.5, such as equal to or less than 2.0 ± 0.5, such as equal to or less than 2.2 ± 0.5, such as equal to or less than 2.5 ± 0.5, the subject is classified as having or likely to have RDS. Preferably, the control ratio is 2.0 ± 0.5, 2.5 ± 0.5 or 3.0 ± 0.5. In other embodiments, if the L/S ratio in the lamellar bodies as measured by the methods disclosed herein is equal to or less than 2.5 ± 0.5, such as equal to or less than 2.6 ± 0.5, such as equal to or less than 2.7 ± 0.5, such as equal to or less than 2.8 ± 0.5, such as equal to or less than 2.9 ± 0.5, such as equal to or less than 3.0 ± 0.5, the subject is classified as having or likely to have RDS. In some embodiments, if the L/S ratio in the lamellar bodies as measured by the methods disclosed herein is less than 1.0 ± 0.5, such as less than 1.2 ± 0.5, such as less than 1.5 ± 0.5, such as less than 1.7 ± 0.5, such as less than 2.0 ± 0.5, such as less than 2.2 ± 0.5, such as less than 2.5 ± 0.5, the subject is classified as having or likely to have RDS. Preferably, the control ratio is 2.0 ± 0.5, 2.5 ± 0.5 or 3.0 ± 0.5. In other embodiments, if the L/S ratio in the lamellar bodies as measured by the methods disclosed herein is equal to or less than 2.5 ± 0.5, such as equal to or less than 2.6 ± 0.5, such as equal to or less than 2.7 ± 0.5, such as equal to or less than 2.8 ± 0.5, such as equal to or less than 2.9 ± 0.5, such as equal to or less than 3.0 ± 0.5, the subject is classified as having or likely to have RDS. In some embodiments, if the L/S ratio in the lamellar bodies as measured by the methods disclosed herein is less than 2.5 ± 0.5, such as less than 2.6 ± 0.5, such as less than 2.7 ± 0.5, such as less than 2.8 ± 0.5, such as less than 2.9 ± 0.5, such as less than 3.0 ± 0.5, the subject is classified as having or likely to have RDS.

The present methods preferably have a specificity of 50 or more, such as 60 or more, such as 70 or more, such as 80 or more, such as 90 or more. The present methods preferably have a sensitivity of 50 or more, such as 60 or more, such as 70 or more, such as 80 or more, such as 90 or more.

The present methods may be performed fast, and are thus well suited for point-of-care units. In some embodiments, the time-to-result of the method is between 5 and 60 minutes, such as between 10 and 50 minutes, such as between 20 and 40 minutes, such as between 25 and 35 minutes, such as about 30 minutes. In some embodiments, the time-to-result of the method is 60 minutes or less, such as 55 minutes or less, such as 50 minutes or less, such as 45 minutes or less, such as 40 minutes or less, such as 35 minutes or less, such as 30 minutes or less, such as 25 minutes or less, such as 20 minutes or less, such as 15 minute or less, such as 10 minutes or less, such as 5 minutes or less.

The time-to-result is herein defined as the time between steps i) and step vi) of the methods.

In some embodiments, only the concentration of lecithin or saturated lecithin is determined and compared to a control value as described above. In other embodiments, the concentration of lecithin or saturated lecithin and the concentration of sphingomyelin are determined, the L/S ratio is calculated and compared to a control ratio as described above. In some embodiments, the concentration of lecithin or saturated lecithin is determined and compared to a control value and the concentration of sphingomyelin is also determined to calculate the L/S ratio which is compared to a control ratio.

### Method of treatment of RDS

Any of the embodiments disclosed herein, i.e. any of the methods described above, may further include a step of treating a subject classified as having or likely to have RDS. In some embodiments, the treatment is administration of a therapeutically effective amount of surfactant to the subject. Preferably, the subject is a newborn individual.

Accordingly, also disclosed herein is a method of treatment of Respiratory Distress Syndrome (RDS) in a newborn individual, comprising the steps of:
i) providing a sample, such as an amniotic fluid sample, a blood sample, an oropharyngeal secretion sample or a gastric aspirate sample, from said newborn individual;
ii) diluting and homogenising said sample in a first volume of a first solution, thereby obtaining a homogenous sample;
iii) centrifuging the homogenous sample to obtain a pellet comprising lamellar bodies and a supernatant;
iv) discarding the supernatant and resuspending the pellet in a second volume of a second solution, thereby obtaining a sample for analysis;
v) determining the amount of a first compound selected from lecithin and saturated lecithin and optionally the amount of sphingomyelin in the sample for analysis, using analysis means;
vi) obtaining a concentration of the first compound and optionally a ratio between the first compound and sphingomyelin;
vii) correlating the concentration of vi) with a control value and optionally correlating the ratio of vi) with a control ratio, wherein a concentration differing from the control value and optionally a ratio differing from the control ratio is indicative of Respiratory Distress Syndrome (RDS) of the subject;
viii) if the concentration of vi) is equal to or less than the control value and/or if the ratio of vi) is equal to or less than the control ratio, administering a therapeutically effective amount of surfactant to the newborn individual.

Also disclosed herein is a method of treatment of Respiratory Distress Syndrome (RDS) in a newborn individual, comprising the steps of:
i) providing a gastric aspirate sample from said newborn individual;
ii) diluting and homogenising said sample in a first volume of a first solution, thereby obtaining a homogenous sample;
iii) centrifuging the homogenous sample to obtain a pellet comprising lamellar bodies and a supernatant;
iv) discarding the supernatant and resuspending the pellet in a second volume of a second solution, thereby obtaining a sample for analysis;
v) determining the amount of a first compound selected from lecithin and saturated lecithin and the amount of sphingomyelin in the sample for analysis, using analysis means;
vi) obtaining a ratio between the first compound and sphingomyelin;
vii) correlating the ratio of vi) with a control ratio, wherein a ratio differing from the control ratio is indicative of Respiratory Distress Syndrome (RDS) of the subject;
viii) if the ratio of vi) is less than the control ratio, administering a therapeutically effective amount of surfactant to the newborn individual.

The control value and the control ratio are as defined in the section entitled "diagnostic" above.

Based on the diagnostic methods outlined herein above a rapid treatment of the individual in need thereof can be achieved, thus resulting in improved survival rate of the individual. The invention is in particular well suited for diagnosing and treating a newborn such as a premature infant.

### Computer implemented method and systems for diagnosing RDS

In one aspect, the invention concerns a computer implemented method for diagnosing RDS based on spectral data acquired from a sample, wherein the sample is a gastric aspirate sample, a blood sample, an orpharyngeal sample or an amniotic fluid samples obtained from a subject, the method comprising the steps of:
i) determining the activity and/or concentration of a first compound selected from lecithin and saturated lecithin and optionally of sphingomyelin in said sample by acquiring spectral data for the sample,
ii) optionally calculating a ratio between the activities and/or concentrations of sphingomyelin and of the first compound,
iii) correlating said concentration with a control value and optionally correlating said ratio with a control ratio, wherein a concentration equal to or lower than the control value and/or a ratio equal to or lower than the control ratio is indicative of RDS of the subject.

In another aspect, the invention concerns a computer implemented method for diagnosing RDS based on spectral data acquired from a sample, wherein the sample is a gastric aspirate sample, an oropharyngeal sample or an amniotic fluid samples obtained from a subject, the method comprising the steps of:
i) determining the activity and/or concentration of sphingomyelin and of a first compound selected from lecithin and saturated lecithin in said sample by acquiring spectral data for the sample,
ii) calculating a ratio between the activities and/or concentrations of sphingomyelin and of the first compound,
iii) correlating said ratio with a control ratio, wherein a ratio differing from the control ratio is indicative of RDS of the subject.

Steps i), ii) and iii) are performed by any of the methods described herein above.

The control value and the control ratio are as defined in the section entitled "diagnostic" above.

As time may be an important factor for treating RDS, the computer-implemented diagnosis method may advantageously be integrated in a diagnosis system that can be installed in hospital departments, such as the neonatal department, preferably in the delivery room. Such a system can integrate spectroscopy, analysis and disease indication that may provide a diagnostic within minutes after a biological sample has been obtained.

Thus, the present method may be integrated in a personal computer or it may be effectuated from a website, mobile phone, smartphone or other electronic device capable of executing computer code. A further embodiment of the invention therefore relates to a computer program product having a computer readable medium, said computer program product being suitable for diagnosing a respiratory disease of a subject based on spectral data acquired from a sample obtained from said subject, said computer program product comprising means for carrying out all the steps of the herein disclosed method, wherein the sample is a gastric aspirate sample, a blood sample, an amniotic fluid sample or an oropharyngeal secretion sample.

In one embodiment, the sample is a gastric aspirate sample. In another embodiment, the sample is an amniotic fluid sample. In another embodiment, the sample is an oropharyngeal secretion. In another embodiment, the sample is a blood sample.

The system may be part of a health monitoring system as described in WO 2008/019695 disclosing a health monitoring service based on a central server, wherein the measurement of the samples is carried out as a local measurement and the measurement data are subsequently sent to a central server, where the data are processed and analysed, for example by expert knowledge systems, and a health profile is generated and sent back to the local system. Thus, the processing means may be fully or partly integrated in a central service remote from the local hospital department or even remote from the hospital. However, the processing means may also be fully integrated in the local system such that the system located in the hospital department includes spectrometer, spectral analysis and processing and disease indication.

### References

1. Dorland's Medical Dictionary - "Neonatal respiratory distress syndrome"
2. Rodriguez RJ, Martin RJ, and Fanaroff, AA. (2002) Neonatal-perinatal medicine: Diseases of the fetus and infant; 7th ed. (2002):1001-1011. St. Louis: Mosby.
3. Kamper J, Wulff K, Larsen C, Lindequist S. (1993) Acta Paediatr;82:193-197.
4. Polin RA, Sahni R. (2002) Semin Neonatol 7:739-789.
5. Verder H. (2007) Acta Paediatr 96:482-484.
6. Verder H, Albertsen P, Ebbesen F, Greisen G, Robertson B, Bertelsen A, Agertoft L, Djernes B, Nathan E, Reinholdt J. (1999) Pediatrics 103:e24.
7. Sandri F, Plavka R, Ancora G, Simeoni U, Stranak Z, Martinelli S, Mosca F, Nona J, Thomson, M, Verder H, Fabbri L, Halliday H. (2010) Pediatrics 125:e140.
8. Bevilacqua G, Parmagiani S, Robertson B. (1996) J Perinat Med 24:1-12.
9. Verder H., "Praenatal bestemmelse af lungematuriteten og forebyggelse af idiopatisk respiratory distress syndrom. Lecithinsphingomyelin ratio i amnionv 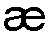 sken" Doctoral dissertation 27 November 1980 at University of Copenhagen.
10. Soll RF. (1999) Cochrane Database Syst Rev 4:CD001456.
11. Stevens TP, Blennow M, Meyers EH, Soll R. (2007) Cochrane Database Syst Rev 2007;4: CD003063.
12. Verder H, Robertson B, Greisen G, Ebbesen F, Albertsen P, Lundstrøm K, Jacobsen T. (1994) N Engl J Med 331:1051-1055.
13. Soll RF. (2012) Neonatology 102:169-171.
14. Van Kaam AH, Jaegere AP, Borensztajn D, Rimensberger PC (2011) Neonatology 100:71-77.
15. Liu K-Z, Dembinski TC, Mantsch HH (1998) Prenatal Diagnosis 18: 1267-1275
16. Verder H, Heiring C, Clark H, Sweet D, Jessen TE, Ebbesen F, Björklund LJ, Andreasson B, Bender L, Bertelsen A, Dahl M, Eschen C, Fenger-Grøn J, Hoffmann SF, Hoskuldsson A, Brussgaard-Mouritsen M, Lundberg F, Postle AD, Schousboe P, Schmidt P, Stanchev H, Sørensen L (2017) Acta Paediatr. 2017 Mar;106(3):430-437

### Examples

A diagnostic test for lung maturity for optimal treatment of respiratory distress syndrome (RDS) has previously been developed based on mid-infrared spectroscopy on gastric aspirates (GAS) [16]. The study was based on analyses of lecithin/sphingomyelin (SM) raiot (L/S) on frozen and thawed GAS. Lecithin was measured as dipalmitoylphosphatidylcholine (DPPC).

In the present study, analyses were performed on fresh GAS. The spectroscopy signal has been enhanced by concentrating the surfactant and problems with interfering proteins, salts and mucus-like, flocculent protein cloths have been avoided.

The method is based on FTIR technology to analyse the contents of precipitated lamellar bodies. Stable measurements by dry transmission require a short path length for the infrared beam passing through the sample. The method if thus focused on removing irrelevant and excess material such as proteins and salts, resulting in improving purity of the lamellar bodies to be analysed.

### Methods

GAS obtained immediately after birth were stored at 4°C and analysed; some samples were analysed immediately, some were analysed within a few hours, others a few days, with a maximal storage of 2 weeks.

The L/S algorithm was built on 85 GAS (DPPC (55 samples) and SM (85 samples)) obtained from infants with gestational age 24-36 weeks. Sampling for FTIR and reference samples were obtained by standard methods. 200 µL GAS were diluted 4 fold with water and centrifuged at 4000 g for 4 minutes. After removal of the supernatant, the samples were resuspended in 100 µL of water and split in 2 aliquots of 50 µL. One aliquot was analysed by FTIR, and one aliquot was analysed by mass spectrocscopy (MS) for measuring contents in phosphatidylcholine (PC) and sphingomyelin. MS was performed as described in [16].

Dry transmission of samples was performed on CaF₂ windows (1 mm thick, 13 mm in diameter, Chrystran). The 50 µL samples were applied onto the CaF₂ windows dried on a hotplate (90°C). The FTIR measurements were performed with Bruker Tensor 27, equipped with a DTGS detector (60 scans, resolution 4 cm⁻¹).

Pellets from treated samples were fixated in 4% paraformaldehyde until preparation for electron microscopy scanning.

### Results

### Viscosity of frozen and fresh samples

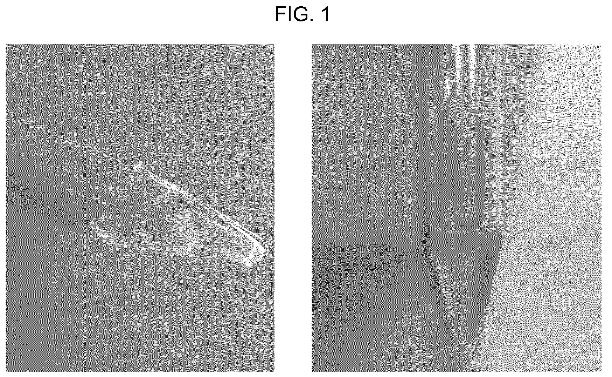

Fresh, frozen and thawed GAS were compared in 30 cases. The mucus-like, flocculent material composed of phospholipids, proteins and mucus appeared mainly to be a consequence of freezing and was observed in both the frozen and the thawed material. These clot-like structures were mostly insoluble in contrast to fresh gastric aspirates which despite a high viscosity did not display clot formation, and could be dissolved and diluted (figure 1).

### Mass spectroscopy of proteins and phospholipids

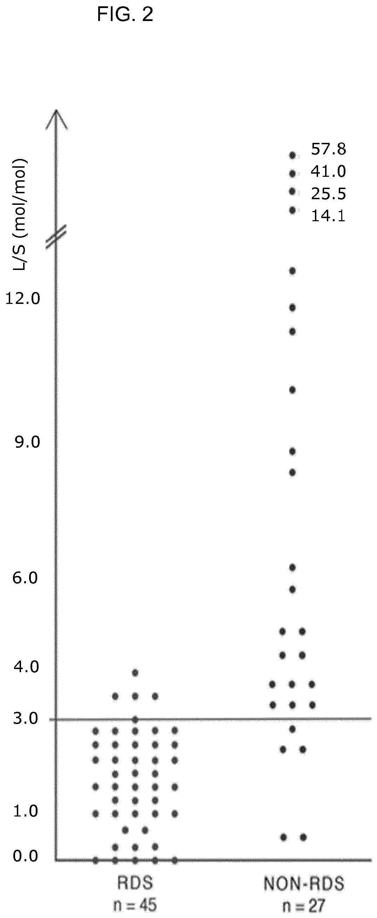

MS of proteins and phospholipids revealed that the mucus-like, flocculent material was consisting of a wide range of proteins and phospholipids. Protein content showed that mucus-like was dominant in the GAS. Further analysis also showed a high concentration of phospholipids. However, analysis of crude GAS and purified LB fractions analysed by MS show high correlations (figure 2).

### Stability of phospholipids during storage

Four fresh GAS from newborns with various gestational age were included. The PC and SM contents were measured by MS at birth and again after storage for four weeks at 4-5°C. The phospholipids were stable and unchanged during the period.

### Electron microscopy

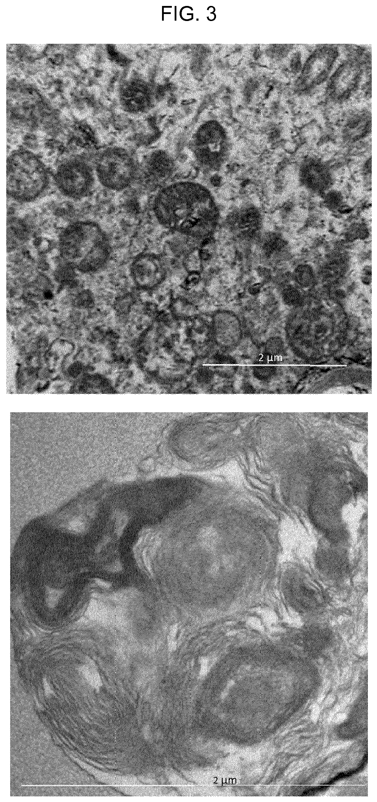

The lamellar bodies were visualized by electron microscopy (figure 3). Pellets obtained from GAS diluted with water showed lamellar body structures in samples from neonates at various gestational ages.

### Sensitivity and specificity

The present method shows a sensitivity of 91% and a specificity of 79% based on 72 neonate GAS. By comparison, diagnosis on a cut-off value (control ratio) for the L/S ratio of 3.0 and DPPC contents alone has a sensitivity of 93% and a specificity of 74%.

### Blood samples

The method was also applied to blood samples. The present method appears to reduce uncertainties as the blood cells during the hypotonic conditions burst and are removed along with the supernatant. MS of phospholipids indicated that most PC and SM originate from lamellar bodies.

### Conclusion

We have developed a method for use with dry transmission that reduces salt and protein contents in the samples, thus resulting in stable and reliable measurements. Dilution of the samples lowers the viscosity of the allowing lamellar bodies to be precipitated by centrifugation at low g-force, where most cellular debris, proteins and salts remain in the supernatant. These improvements leave a smaller amount of more relevant material in the form of lamellar bodies carrying the surfactant. Water is evaporated by drying the samples, for example on a hotplate. Furthermore, the method is temperature independent, at least in the range of 20 to 40°C.

Using an appropriate spectroscope, lung maturity can thus be measured and determined within the first 10 to 15 minutes of life, with high specificity and sensitivity.

## Claims

1. A method for diagnosing Respiratory Distress Syndrome (RDS) in a subject, the method comprising the steps of:
i) using a sample from said subject, wherein the sample is an amniotic fluid sample, a blood sample, an oropharyngeal secretion sample or a gastric aspirate sample, preferably the sample is a gastric aspirate sample or an oropharyngeal secretion, for;
ii) homogenising and diluting said sample in a first volume of a first solution, thereby obtaining a homogenous sample;
iii) centrifuging the sample of step ii) to obtain a pellet comprising lamellar bodies, and a supernatant;
iv) discarding the supernatant and resuspending the pellet in a second volume of a second solution, thereby obtaining a sample for analysis;
v) determining the amount of a first compound selected from lecithin and saturated lecithin and optionally the amount of sphingomyelin in the sample for analysis, using analysis means;
vi) obtaining a concentration of the first compound and/or a ratio between the first compound and sphingomyelin;
vii) correlating the concentration of vi) with a control value, wherein a concentration equal to or lower than the control value is indicative of the subject having Respiratory Distress Syndrome (RDS); and/or correlating the ratio of vi) with a control ratio, wherein a ratio equal to or lower than the control ratio is indicative of the subject having Respiratory Distress Syndrome (RDS).

2. The method according to any one of the preceding claims, wherein the sample has a volume between 10 and 1000 µL, such as between 10 and 750 µL, such as between 20 and 500 µL, such as between 30 and 250 µL, such as between 40 and 125 µL, such as between 50 and 100 µL, such as between 60 and 90 µL, such as between 70 and 80 µL, such as 50 µL, 75 µL or 100 µL.

3. The method according to any one of the preceding claims, wherein the first solution is a hypotonic solution such as water or deionised water.

4. The method according to any one of the preceding claims, wherein the ratio of the volume of the sample of step i) to the volume of the first solution used in step ii) is between 1:1 and 1:10, such as 1:1, 1:2, 1:3, 1:4, 1:5, 1:6, 1:7, 1:8, 1:9 or 1:10, preferably 1:4 or 1:6.

5. The method according to any one of the preceding claims, wherein the centrifugation of step iii) is performed at a force between 500 and 10000 g, such as 4000 g, for a duration of 1 min to 10 min, such as 2 min to 9 min, such as 3 min to 8 min, such as 4 min to 7 min, such as 5 min to 6 min, such as 4 min, 5 min or 6 min.

6. The method according to any one of the preceding claims, wherein the second solution is a hypotonic solution such as water or deionised water, or saline solution.

7. The method according to any one of the preceding claims, wherein the analysis means is selected an infrared spectrometer, such as a Fourier transformed infrared (FTIR) spectrometer.

8. The method according to any one of the preceding claims, further comprising determining the amount of a further compound such as phosphatidylglycerol.

9. The method according to any one of the preceding claims, wherein the time-to-result of the method is between 5 and 60 minutes, such as between 8 and 30 minutes, such as 15 minutes.

10. The method according to any one of the preceding claims, wherein the control ratio is between 1.0 and 3.0 ± 0.5, such as between 1.5 and 2.9± 0.5, such as between 2.0 and 2.8 ± 0.5, such as between 2.2 and 2.7 ± 0.5, such as between 2.4 and 2.6 ± 0.5, such as 2.5 ± 0.5 or 2.0 ± 0.5, or between 2.0 and 5.0 ± 0.5, such as between 2.2 and 4.5 ± 0.5, such as between 2.4 and 4.0 ± 0.5, such as between 2.6 and 3.5 ± 0.5, such as between 2.8 and 3.2 ± 0.5, such as 3 ± 0.5.

11. Surfactant for use in the treatment of Respiratory Distress Syndrome (RDS) in a newborn individual, wherein the surfactant is administered to a subject having an increased likelihood of RDS as determined by the method comprising the steps of:
i) providing a sample from said newborn individual, wherein the sample is an amniotic fluid sample, a blood sample, an oropharyngeal secretion sample or a gastric aspirate sample;
ii) diluting and homogenising said sample in a first volume of a first solution, thereby obtaining a homogenous sample;
iii) centrifuging the homogenous sample to obtain a pellet comprising lamellar bodies and a supernatant;
iv) discarding the supernatant and resuspending the pellet in a second volume of a second solution, thereby obtaining a sample for analysis;
v) determining the amount of a first compound selected from lecithin and saturated lecithin and optionally the amount of sphingomyelin in the sample for analysis, using analysis means;
vi) obtaining a concentration of the first compound and optionally a ratio between the first compound and sphingomyelin; and
vii) correlating the concentration of vi) with a control value, wherein a concentration equal to or lower than the control value is indicative of the subject having Respiratory Distress Syndrome (RDS); and/or correlating the ratio of vi) with a control ratio, wherein a ratio equal to or lower than the control ratio is indicative of the subject having Respiratory Distress Syndrome (RDS),
optionally wherein steps i) to vii) are performed as defined in any one of claims 2 to 10.

12. The surfactant for use according to claim 11, wherein the control ratio is between 1.0 and 3.0 ± 0.5, such as between 1.5 and 2.9± 0.5, such as between 2.0 and 2.8 ± 0.5, such as between 2.2 and 2.7 ± 0.5, such as between 2.4 and 2.6 ± 0.5, such as 2.5 ± 0.5 or 2.0 ± 0.5, or between 2.0 and 5.0 ± 0.5, such as between 2.2 and 4.5 ± 0.5, such as between 2.4 and 4.0 ± 0.5, such as between 2.6 and 3.5 ± 0.5, such as between 2.8 and 3.2 ± 0.5, such as 3 ± 0.5.

13. A computer implemented method for diagnosing RDS based on spectral data acquired from a sample obtained from a subject, wherein the sample is an amniotic fluid sample, a blood sample, an oropharyngeal secretion sample or a gastric aspirate sample, the method comprising the steps of:
i) determining the activity and/or concentration of a first compound selected from lecithin and saturated lecithin and optionally of sphingomyelin in said sample by acquiring spectral data for the sample,
ii) optionally calculating a ratio between the activities and/or concentrations of sphingomyelin and of the first compound,
iii) correlating said concentration with a control value and optionally correlating said ratio with a control ratio, wherein a concentration equal to or lower than the control value and/or a ratio equal to or lower than the control ratio is indicative of RDS of the subject
wherein the method further comprises the features of any of claims 1 to 10.

14. A computer program product having a computer readable medium, said computer program product suitable for diagnosing RDS of a subject based on spectral data acquired from a sample obtained from said subject, wherein the sample is an amniotic fluid sample, a blood sample, an oropharyngeal secretion sample or a gastric aspirate sample, said computer program product comprising means for carrying out all the steps of the method as defined in claim 13.

## Patentansprüche

1. Verfahren zur Diagnose des Atemnotsyndroms (RDS) bei einem Subjekt, wobei das Verfahren die folgenden Schritte umfasst:
i) Verwenden einer Probe von dem Subjekt, wobei die Probe eine Fruchtwasserprobe, eine Blutprobe, eine oropharyngeale Sekretprobe oder eine Magenaspiratprobe ist, wobei die Probe vorzugsweise eine Magenaspiratprobe oder ein oropharyngeales Sekret ist, zum;
ii) Homogenisieren und Verdünnen der Probe in einem ersten Volumen einer ersten Lösung, wodurch eine homogene Probe erhalten wird;
iii) Zentrifugieren der Probe aus Schritt ii), um ein Pellet, das Lamellarkörper umfasst, und einen Überstand zu erhalten;
iv) Verwerfen des Überstands und Resuspendieren des Pellets in einem zweiten Volumen einer zweiten Lösung, wodurch eine Probe zur Analyse erhalten wird;
v) Bestimmen der Menge einer ersten Verbindung, ausgewählt aus Lecithin und gesättigtem Lecithin, und gegebenenfalls der Menge an Sphingomyelin in der Probe zur Analyse unter Verwendung von Analysemitteln;
vi) Erhalten einer Konzentration der ersten Verbindung und/oder eines Verhältnisses zwischen der ersten Verbindung und Sphingomyelin;
vii) Korrelieren der Konzentration aus vi) mit einem Kontrollwert, wobei eine Konzentration gleich dem oder niedriger als der Kontrollwert darauf hinweist, dass das Subjekt ein Atemnotsyndrom (RDS) aufweist; und/oder Korrelieren des Verhältnisses aus vi) mit einem Kontrollverhältnis, wobei ein Verhältnis gleich dem oder niedriger als das Kontrollverhältnis darauf hinweist, dass das Subjekt ein Atemnotsyndrom (RDS) aufweist.

2. Verfahren nach einem der vorhergehenden Ansprüche, wobei die Probe ein Volumen zwischen 10 und 1000 µL aufweist, wie etwa zwischen 10 und 750 µL, wie etwa zwischen 20 und 500 µL, wie etwa zwischen 30 und 250 µL, wie etwa zwischen 40 und 125 µL, wie etwa zwischen 50 und 100 µL, wie etwa zwischen 60 und 90 µL, wie etwa zwischen 70 und 80 µL, wie etwa 50 µL, 75 µL oder 100 µL.

3. Verfahren nach einem der vorhergehenden Ansprüche, wobei die erste Lösung eine hypotonische Lösung wie etwa Wasser oder deionisiertes Wasser ist.

4. Verfahren nach einem der vorhergehenden Ansprüche, wobei das Verhältnis des Volumens der Probe aus Schritt i) zu dem Volumen der in Schritt ii) verwendeten ersten Lösung zwischen 1:1 und 1:10 liegt, wie etwa 1:1, 1:2, 1:3, 1:4, 1:5, 1:6, 1:7, 1:8, 1:9 oder 1:10, vorzugsweise 1:4 oder 1:6.

5. Verfahren nach einem der vorhergehenden Ansprüche, wobei die Zentrifugation aus Schritt iii) bei einer Kraft zwischen 500 und 10000 g, wie etwa 4000 g, für eine Dauer von 1 min bis 10 min, wie etwa 2 min bis 9 min, wie etwa 3 min bis 8 min, wie etwa 4 min bis 7 min, wie etwa 5 min bis 6 min, wie etwa 4 min, 5 min oder 6 min, durchgeführt wird.

6. Verfahren nach einem der vorhergehenden Ansprüche, wobei die zweite Lösung eine hypotonische Lösung wie etwa Wasser oder deionisiertes Wasser, oder eine Salzlösung ist.

7. Verfahren nach einem der vorhergehenden Ansprüche, wobei das Analysemittel ausgewählt ist aus einem Infrarotspektrometer, wie etwa einem Fourier-Transformations-Infrarot(FTIR)-Spektrometer.

8. Verfahren nach einem der vorhergehenden Ansprüche, ferner umfassend das Bestimmen der Menge einer weiteren Verbindung wie etwa Phosphatidylglycerin.

9. Verfahren nach einem der vorhergehenden Ansprüche, wobei die Zeit bis zum Ergebnis des Verfahrens zwischen 5 und 60 Minuten, wie etwa zwischen 8 und 30 Minuten, wie etwa 15 Minuten, beträgt.

10. Verfahren nach einem der vorhergehenden Ansprüche, wobei das Kontrollverhältnis zwischen 1,0 und 3,0 ± 0,5 liegt, wie etwa zwischen 1,5 und 2,9± 0,5, wie etwa zwischen 2,0 und 2,8 ± 0,5, wie etwa zwischen 2,2 und 2,7 ± 0,5, wie etwa zwischen 2,4 und 2,6 ± 0,5, wie etwa 2,5 ± 0,5 oder 2,0 ± 0,5, oder zwischen 2,0 und 5,0 ± 0,5, wie etwa zwischen 2,2 und 4,5 ± 0,5, wie etwa zwischen 2,4 und 4,0 ± 0,5, wie etwa zwischen 2,6 und 3,5 ± 0,5, wie etwa zwischen 2,8 und 3,2 ± 0,5, wie etwa 3 ± 0,5.

11. Surfactant zur Verwendung bei der Behandlung des Atemnotsyndroms (RDS) bei einem neugeborenen Individuum, wobei der Surfactant einem Subjekt verabreicht wird, das eine erhöhte Wahrscheinlichkeit für RDS aufweist, wie durch das Verfahren bestimmt, das die folgenden Schritte umfasst:
i) Bereitstellen einer Probe von dem neugeborenen Individuum, wobei die Probe eine Fruchtwasserprobe, eine Blutprobe, eine oropharyngeale Sekretprobe oder eine Magenaspiratprobe ist;
ii) Verdünnen und Homogenisieren der Probe in einem ersten Volumen einer ersten Lösung, wodurch eine homogene Probe erhalten wird;
iii) Zentrifugieren der homogenen Probe, um ein Pellet, das Lamellarkörper umfasst, und einen Überstand zu erhalten;
iv) Verwerfen des Überstands und Resuspendieren des Pellets in einem zweiten Volumen einer zweiten Lösung, wodurch eine Probe zur Analyse erhalten wird;
v) Bestimmen der Menge einer ersten Verbindung, ausgewählt aus Lecithin und gesättigtem Lecithin, und gegebenenfalls der Menge an Sphingomyelin in der Probe zur Analyse unter Verwendung von Analysemitteln;
vi) Erhalten einer Konzentration der ersten Verbindung und gegebenenfalls eines Verhältnisses zwischen der ersten Verbindung und Sphingomyelin; und
vii) Korrelieren der Konzentration aus vi) mit einem Kontrollwert, wobei eine Konzentration gleich dem oder niedriger als der Kontrollwert darauf hinweist, dass das Subjekt ein Atemnotsyndrom (RDS) aufweist; und/oder Korrelieren des Verhältnisses aus vi) mit einem Kontrollverhältnis, wobei ein Verhältnis gleich dem oder niedriger als das Kontrollverhältnis darauf hinweist, dass das Subjekt ein Atemnotsyndrom (RDS) aufweist,
wobei gegebenenfalls die Schritte i) bis vii) wie in einem der Ansprüche 2 bis 10 definiert durchgeführt werden.

12. Surfactant zur Verwendung nach Anspruch 11, wobei das Kontrollverhältnis zwischen 1,0 und 3,0 ± 0,5 liegt, wie etwa zwischen 1,5 und 2,9± 0,5, wie etwa zwischen 2,0 und 2,8 ± 0,5, wie etwa zwischen 2,2 und 2,7 ± 0,5, wie etwa zwischen 2,4 und 2,6 ± 0,5, wie etwa 2,5 ± 0,5 oder 2,0 ± 0,5, oder zwischen 2,0 und 5,0 ± 0,5, wie etwa zwischen 2,2 und 4,5 ± 0,5, wie etwa zwischen 2,4 und 4,0 ± 0,5, wie etwa zwischen 2,6 und 3,5 ± 0,5, wie etwa zwischen 2,8 und 3,2 ± 0,5, wie etwa 3 ± 0,5.

13. Computerimplementiertes Verfahren zur Diagnose von RDS auf der Grundlage von Spektraldaten, die von einer von einem Subjekt erhaltenen Probe erfasst wurden, wobei die Probe eine Fruchtwasserprobe, eine Blutprobe, eine oropharyngeale Sekretprobe oder eine Magenaspiratprobe ist, wobei das Verfahren die folgenden Schritte umfasst:
i) Bestimmen der Aktivität und/oder Konzentration einer ersten Verbindung, ausgewählt aus Lecithin und gesättigtem Lecithin, und gegebenenfalls von Sphingomyelin in der Probe durch Erfassen von Spektraldaten für die Probe,
ii) gegebenenfalls Berechnen eines Verhältnisses zwischen den Aktivitäten und/oder Konzentrationen von Sphingomyelin und der ersten Verbindung,
iii) Korrelieren der Konzentration mit einem Kontrollwert und gegebenenfalls Korrelieren des Verhältnisses mit einem Kontrollverhältnis, wobei eine Konzentration gleich dem oder niedriger als der Kontrollwert und/oder ein Verhältnis gleich dem oder niedriger als das Kontrollverhältnis auf RDS des Subjekts hinweist
wobei das Verfahren ferner die Merkmale eines der Ansprüche 1 bis 10 umfasst.

14. Computerprogrammprodukt mit einem computerlesbaren Medium, wobei das Computerprogrammprodukt zur Diagnose von RDS eines Subjekts auf der Grundlage von Spektraldaten geeignet ist, die von einer von dem Subjekt erhaltenen Probe erfasst wurden, wobei die Probe eine Fruchtwasserprobe, eine Blutprobe, eine oropharyngeale Sekretprobe oder eine Magenaspiratprobe ist, wobei das Computerprogrammprodukt Mittel zum Ausführen aller Schritte des in Anspruch 13 definierten Verfahrens umfasst.

## Revendications

1. Procédé de diagnostic du syndrome de détresse respiratoire (RDS) chez un sujet, le procédé comprenant les étapes consistant à :
i) utiliser un échantillon provenant dudit sujet, dans lequel l'échantillon est un échantillon de liquide amniotique, un échantillon de sang, un échantillon de sécrétion oropharyngée ou un échantillon d'aspirat gastrique, de préférence l'échantillon est un échantillon d'aspirat gastrique ou une sécrétion oropharyngée, pour ;
ii) homogénéiser et diluer ledit échantillon dans un premier volume d'une première solution, obtenant ainsi un échantillon homogène ;
iii) centrifuger l'échantillon de l'étape ii) pour obtenir un culot comprenant des corps lamellaires, et un surnageant ;
iv) éliminer le surnageant et remettre le culot en suspension dans un second volume d'une seconde solution, obtenant ainsi un échantillon pour analyse ;
v) déterminer la quantité d'un premier composé choisi parmi la lécithine et la lécithine saturée et éventuellement la quantité de sphingomyéline dans l'échantillon pour analyse, à l'aide de moyens d'analyse ;
vi) obtenir une concentration du premier composé et/ou un rapport entre le premier composé et la sphingomyéline ;
vii) corréler la concentration de vi) avec une valeur témoin, dans lequel une concentration égale ou inférieure à la valeur témoin indique que le sujet présente un syndrome de détresse respiratoire (RDS) ; et/ou corréler le rapport de vi) avec un rapport témoin, dans lequel un rapport égal ou inférieur au rapport témoin indique que le sujet présente un syndrome de détresse respiratoire (RDS).

2. Procédé selon l'une quelconque des revendications précédentes, dans lequel l'échantillon a un volume compris entre 10 et 1000 uL, tel qu'entre 10 et 750 uL, tel qu'entre 20 et 500 uL, tel qu'entre 30 et 250 uL, tel qu'entre 40 et 125 uL, tel qu'entre 50 et 100 uL, tel qu'entre 60 et 90 uL, tel qu'entre 70 et 80 uL, tel que 50 uL, 75 uL ou 100 uL.

3. Procédé selon l'une quelconque des revendications précédentes, dans lequel la première solution est une solution hypotonique telle que de l'eau ou de l'eau désionisée.

4. Procédé selon l'une quelconque des revendications précédentes, dans lequel le rapport du volume de l'échantillon de l'étape i) au volume de la première solution utilisée à l'étape ii) est compris entre 1:1 et 1:10, tel que 1:1, 1:2, 1:3, 1:4, 1:5, 1:6, 1:7, 1:8, 1:9 ou 1:10, de préférence 1:4 ou 1:6.

5. Procédé selon l'une quelconque des revendications précédentes, dans lequel la centrifugation de l'étape iii) est effectuée à une force comprise entre 500 et 10000 g, telle que 4000 g, pendant une durée de 1 min à 10 min, telle que 2 min à 9 min, telle que 3 min à 8 min, telle que 4 min à 7 min, telle que 5 min à 6 min, telle que 4 min, 5 min ou 6 min.

6. Procédé selon l'une quelconque des revendications précédentes, dans lequel la seconde solution est une solution hypotonique telle que de l'eau ou de l'eau désionisée, ou une solution saline.

7. Procédé selon l'une quelconque des revendications précédentes, dans lequel les moyens d'analyse sont choisis parmi un spectromètre infrarouge, tel qu'un spectromètre infrarouge à transformée de Fourier (FTIR).

8. Procédé selon l'une quelconque des revendications précédentes, comprenant en outre la détermination de la quantité d'un composé supplémentaire tel que le phosphatidylglycérol.

9. Procédé selon l'une quelconque des revendications précédentes, dans lequel le délai d'obtention du résultat du procédé est compris entre 5 et 60 minutes, tel qu'entre 8 et 30 minutes, tel que 15 minutes.

10. Procédé selon l'une quelconque des revendications précédentes, dans lequel le rapport témoin est compris entre 1.0 et 3.0 ± 0.5, tel qu'entre 1.5 et 2.9± 0.5, tel qu'entre 2.0 et 2.8 ± 0.5, tel qu'entre 2.2 et 2.7 ± 0.5, tel qu'entre 2.4 et 2.6 ± 0.5, tel que 2.5 ± 0.5 ou 2.0 ± 0.5, ou entre 2.0 et 5.0 ± 0.5, tel qu'entre 2.2 et 4.5 ± 0.5, tel qu'entre 2.4 et 4.0 ± 0.5, tel qu'entre 2.6 et 3.5 ± 0.5, tel qu'entre 2.8 et 3.2 ± 0.5, tel que 3 ± 0.5.

11. Tensioactif pour utilisation dans le traitement du syndrome de détresse respiratoire (RDS) chez un nouveau-né, dans lequel le tensioactif est administré à un sujet présentant une probabilité accrue de RDS telle que déterminée par le procédé comprenant les étapes consistant à :
i) fournir un échantillon provenant dudit nouveau-né, dans lequel l'échantillon est un échantillon de liquide amniotique, un échantillon de sang, un échantillon de sécrétion oropharyngée ou un échantillon d'aspirat gastrique ;
ii) diluer et homogénéiser ledit échantillon dans un premier volume d'une première solution, obtenant ainsi un échantillon homogène ;
iii) centrifuger l'échantillon homogène pour obtenir un culot comprenant des corps lamellaires et un surnageant ;
iv) éliminer le surnageant et remettre le culot en suspension dans un second volume d'une seconde solution, obtenant ainsi un échantillon pour analyse ;
v) déterminer la quantité d'un premier composé choisi parmi la lécithine et la lécithine saturée et éventuellement la quantité de sphingomyéline dans l'échantillon pour analyse, à l'aide de moyens d'analyse ;
vi) obtenir une concentration du premier composé et éventuellement un rapport entre le premier composé et la sphingomyéline ; et
vii) corréler la concentration de vi) avec une valeur témoin, dans lequel une concentration égale ou inférieure à la valeur témoin indique que le sujet présente un syndrome de détresse respiratoire (RDS) ; et/ou corréler le rapport de vi) avec un rapport témoin, dans lequel un rapport égal ou inférieur au rapport témoin indique que le sujet présente un syndrome de détresse respiratoire (RDS),
éventuellement dans lequel les étapes i) à vii) sont effectuées telles que définies dans l'une quelconque des revendications 2 à 10.

12. Tensioactif pour utilisation selon la revendication 11, dans lequel le rapport témoin est compris entre 1.0 et 3.0 ± 0.5, tel qu'entre 1.5 et 2.9± 0.5, tel qu'entre 2.0 et 2.8 ± 0.5, tel qu'entre 2.2 et 2.7 ± 0.5, tel qu'entre 2.4 et 2.6 ± 0.5, tel que 2.5 ± 0.5 ou 2.0 ± 0.5, ou entre 2.0 et 5.0 ± 0.5, tel qu'entre 2.2 et 4.5 ± 0.5, tel qu'entre 2.4 et 4.0 ± 0.5, tel qu'entre 2.6 et 3.5 ± 0.5, tel qu'entre 2.8 et 3.2 ± 0.5, tel que 3 ± 0.5.

13. Procédé mis en œuvre par ordinateur pour diagnostiquer le RDS sur la base de données spectrales acquises à partir d'un échantillon obtenu auprès d'un sujet, dans lequel l'échantillon est un échantillon de liquide amniotique, un échantillon de sang, un échantillon de sécrétion oropharyngée ou un échantillon d'aspirat gastrique, le procédé comprenant les étapes consistant à :
i) déterminer l'activité et/ou la concentration d'un premier composé choisi parmi la lécithine et la lécithine saturée et éventuellement de sphingomyéline dans ledit échantillon par acquisition de données spectrales pour l'échantillon,
ii) éventuellement calculer un rapport entre les activités et/ou les concentrations de sphingomyéline et du premier composé,
iii) corréler ladite concentration avec une valeur témoin et éventuellement corréler ledit rapport avec un rapport témoin, dans lequel une concentration égale ou inférieure à la valeur témoin et/ou un rapport égal ou inférieur au rapport témoin indique un RDS du sujet
dans lequel le procédé comprend en outre les caractéristiques de l'une quelconque des revendications 1 à 10.

14. Produit programme d'ordinateur comportant un support lisible par ordinateur, ledit produit programme d'ordinateur étant approprié pour diagnostiquer le RDS d'un sujet sur la base de données spectrales acquises à partir d'un échantillon obtenu auprès dudit sujet, dans lequel l'échantillon est un échantillon de liquide amniotique, un échantillon de sang, un échantillon de sécrétion oropharyngée ou un échantillon d'aspirat gastrique, ledit produit programme d'ordinateur comprenant des moyens pour exécuter toutes les étapes du procédé tel que défini dans la revendication 13.
